# EUROPEAN PATENT APPLICATION

(11) **EP 3 875 589 A1**
(43) Date of publication of application: **08.09.2021**
(21) Application number: 19879891.0
(22) Date of filing: 28.10.2019
(51) Int. Cl.: C12N 15/56, C12N 1/15, C12N 1/19, C12N 1/21, C12N 5/10, C12N 9/28, C12N 15/63, C12P 19/14

(54) **MALTOTRIOSE-GENERATING AMYLASE**

(30) Priority: 31.10.2018 JP 2018204917
(71) Applicant: Amano Enzyme Inc., Nagoya-shi Aichi 460-8630 (JP)
(72) Inventor: ONO, Atsushi, Kakamigahara-shi, Gifu 509-0109 (JP); MATSUBARA, Hirotaka, Kakamigahara-shi, Gifu 509-0109 (JP); KATASE, Toru, Kakamigahara-shi, Gifu 509-0109 (JP)
(74) Representative: Müller-Boré & Partner Patentanwälte PartG mbB
(86) International application number: PCT/JP2019/042168
(87) International publication number: WO 2020/090734

(57) **Abstract**

Provided is a novel enzyme that generates maltotriose. This maltotriose-generating amylase comprises any one of the following polypeptides: a polypeptide of the amino acid sequences represented by SEQ ID NOs. 1-3; a polypeptide of the amino acid sequences represented by SEQ ID NOs. 1-3, with one or more amino acids substituted, added, inserted or deleted, the polypeptide being capable of generating maltotriose; a polypeptide having at least 70% sequence identity with each of the amino acid sequences represented by SEQ ID NOs. 1-3, the polypeptide being capable of generating maltotriose.

## Description

### TECHNICAL FIELD

The present invention relates to a maltotriose-producing amylase.

### BACKGROUND ART

Maltotriose is a sugar, obtained by partially decomposing a starchy material with an enzyme or an acid, in which three glucose molecules are bonded through α-1,4-glucoside bonds. Although the sweetness of maltotriose is about 17% of that of sugar, maltotriose is used as a low-sweetener for foods because of its mellow sweetness. In addition, maltotriose is useful as an anti-drying agent for foods, a crystallization inhibitor for sugar, and an antiaging agent for starch because of its excellent hygroscopicity and water holding property. Furthermore, maltotriose is also useful as a saccharide material used in food processing because of its thermal stability superior to that of glucose and maltose.

Maltotriose can be obtained from a by-product of producing maltose. However, from the viewpoint of stably supplying maltotriose industrially, it is desirable to produce maltotriose by decomposing a starchy material enzymatically. As enzymes that produce maltotriose from a starchy material, N-A468 enzyme derived from Streptomyces griseus (Non-Patent Document 1) and amylase G3 derived from Bacillus subtilis (Non-Patent Document 2) are known. N-A468 enzyme is classified in the same manner as a β-amylase and decomposes a starchy material into maltotriose units, and amylase G3 is classified in the same manner as an α-amylase and produces a starch decomposition product containing maltotriose as a main component.

### PRIOR ART DOCUMENTS

### NON-PATENT DOCUMENTS

Non-Patent Document 1: Journal of the Japanese Society of Starch Science, Vol. 23, No. 3, 175-181 (1979)
Non-Patent Document 2: Agricultural and Biological Chemistry 1985, Vol. 49, Issue 4, 1091-1097

### SUMMARY OF THE INVENTION

### PROBLEMS TO BE SOLVED BY THE INVENTION

However, these enzymes have low production efficiency from microorganisms, and the low production efficiency remains a problem from the viewpoint of stably supplying maltotriose industrially. Therefore, it is desired to obtain a novel enzyme that generates maltotriose.

An object of the present invention is to provide a novel enzyme that generates maltotriose.

### MEANS FOR SOLVING THE PROBLEM

As a result of intensive studies, the present inventors have found a maltotriose-generating amylase derived from a bacterium belonging to the genus Cellulosimicrobium (hereinafter referred to as "maltotriose-producing amylase"). The present invention has been completed on the basis of these findings. That is, the present invention provides the invention of the aspects described below.

Item 1. A maltotriose-producing amylase including a polypeptide selected from the group consisting of:
   (1-1) a polypeptide having an amino acid sequence shown in SEQ ID NO: 1;
   (1-2) polypeptides that have an amino acid sequence with substitution, addition, insertion, or deletion of one or more amino acids in the amino acid sequence shown in SEQ ID NO: 1, and have maltotriose-producing ability;
   (1-3) polypeptides that have a sequence identity to the amino acid sequence shown in SEQ ID NO: 1 of 70% or more, and have maltotriose-producing ability;
   (2-1) a polypeptide having an amino acid sequence shown in SEQ ID NO: 2;
   (2-2) polypeptides that have an amino acid sequence with substitution, addition, insertion, or deletion of one or more amino acids in the amino acid sequence shown in SEQ ID NO: 2, and have maltotriose-producing ability;
   (2-3) polypeptides that have a sequence identity to the amino acid sequence shown in SEQ ID NO: 2 of 70% or more, and have maltotriose-producing ability;
   (3-1) a polypeptide having an amino acid sequence shown in SEQ ID NO: 3;
   (3-2) polypeptides that have an amino acid sequence with substitution, addition, insertion, or deletion of one or more amino acids in the amino acid sequence shown in SEQ ID NO: 3, and have maltotriose-producing ability; and
   (3-3) polypeptides that have a sequence identity to the amino acid sequence shown in SEQ ID NO: 3 of 70% or more, and have maltotriose-producing ability.
Item 2. A DNA encoding the maltotriose-producing amylase according to the item 1.
Item 3. A recombinant vector including the DNA according to the item 2.
Item 4. A transformant obtained by transforming a host with the DNA according to the item 2 or the recombinant vector according to the item 3.
Item 5. A method for producing the maltotriose-producing amylase according to the item 1, the method including the step of culturing the transformant according to the item 4.
Item. 6. An enzyme preparation including the maltotriose-producing amylase according to the item 1.
Item 7. A method for producing maltotriose, the method including the step of producing maltotriose from a starchy material using the maltotriose-producing amylase according to the item 1.

### ADVANTAGES OF THE INVENTION

According to the present invention, a novel enzyme is provided that generates maltotriose.

### BRIEF DESCRIPTION OF THE DRAWINGS

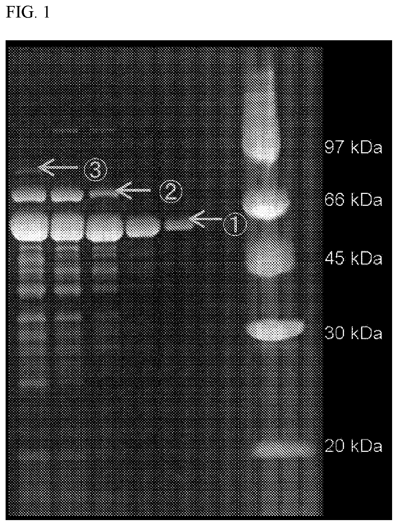

Fig. 1 shows the result of purification by sodium dodecyl sulfate polyacrylamide gel electrophoresis (SDS-PAGE) of an α-amylase having maltotriose-producing activity, obtained from a bacterium belonging to the genus Cellulosimicrobium in Test Example 1.

### EMBODIMENTS OF THE INVENTION

Hereinafter, the present invention will be described in detail. Except for in a sequence listing, 20 kinds of amino acid residues in amino acid sequences may be represented by a one-letter abbreviation. That is, glycine (Gly) is represented by G, alanine (Ala) is A, valine (Val) is V, leucine (Leu) is L, isoleucine (Ile) is I, phenylalanine (Phe) is F, tyrosine (Tyr) is Y, tryptophan (Trp) is W, serine (Ser) is S, threonine (Thr) is T, cysteine (Cys) is C, methionine (Met) is M, aspartic acid (Asp) is D, glutamic acid (Glu) is E, asparagine (Asn) is N, glutamine (Gln) is Q, lysine (Lys) is K, arginine (Arg) is R, histidine (His) is H, and proline (Pro) is P.

In an amino acid sequence described in the present description, the left end indicates the N-terminal, and the right end indicates the C-terminal.

In the present description, "non-polar amino acids" include alanine, valine, leucine, isoleucine, proline, methionine, phenylalanine, and tryptophan. "Non-charged amino acids" include glycine, serine, threonine, cysteine, tyrosine, asparagine, and glutamine. "Acidic amino acids" include aspartic acid and glutamic acid. "Basic amino acids" include lysine, arginine, and histidine.

### 1. Maltotriose-Producing Amylase

The maltotriose-producing amylase of the present invention is derived from a bacterium belonging to the genus Cellulosimicrobium.

The first embodiment of the maltotriose-producing amylase of the present invention is a polypeptide selected from the group consisting of:

(1-1) a polypeptide having an amino acid sequence shown in SEQ ID NO: 1;
(1-2) polypeptides that have an amino acid sequence with substitution, addition, insertion, or deletion of one or more amino acids in the amino acid sequence shown in SEQ ID NO: 1, and have maltotriose-producing ability; and
(1-3) polypeptides that have a sequence identity to the amino acid sequence shown in SEQ ID NO: 1 of 70% or more, and have maltotriose-producing ability.

The polypeptide selected from the group consisting of (1-1) to (1-3) described above has activity that is α-amylase activity to break an α-1,4-bond of a starchy material and produce maltotriose.

The polypeptide described in (1-2) may have an amino acid sequence into which only one modification of substitution, addition, insertion, or deletion (for example, substitution) is introduced, or may have an amino acid sequence into which two or more modifications (for example, substitution and insertion) are introduced. In the polypeptide described in (1-2), the number of amino acids substituted, added, inserted, or deleted is to be one, two or more, or several. For example, the number is 1 to 10, preferably 1 to 8, 1 to 6, 1 to 5, or 1 to 4, more preferably 1 to 3, and particularly preferably 1 or 2, or 1.

The polypeptide described in (1-3) is to have a sequence identity to the amino acid sequence shown in SEQ ID NO: 1 of 70% or more, and the sequence identity is preferably 80% or more, 85% or more, or 90% or more, and more preferably 95% or more, 97% or more, or 98% or more, and particularly preferably 99% or more.

Here, in the polypeptide described in (1-3), the sequence identity to the amino acid sequence shown in SEQ ID NO: 1 is calculated by comparison with the amino acid sequence shown in SEQ ID NO: 1. The term "sequence identity" refers to the value of the identity of amino acid sequences, obtained by bl2seq program (Tatiana A. Tatsusova, Thomas L. Madden, FEMS Microbiol. Lett., Vol. 174, p 247-250, 1999) in BLAST PACKAGE [sgi32 bit edition, Version 2.0.12; available from National Center for Biotechnology Information (NCBI)]. The parameters are to be set to Gap insertion Cost value: 11 and Gap extension Cost value: 1.

In the polypeptide described in (1-2) and (1-3), the amino acids at positions 196, 223, 284, 107, 166, 118, 119, 122, 123, 158, 161, 214, 215, 216, 217, 247, 250, 252, 253, 278, 283, 336, and 342 of the amino acid sequence shown in SEQ ID NO: 1 are considered to contribute to the activity, therefore, it is desirable not to introduce substitution or deletion at these sites. The amino acids at positions 107 and 166 are presumed to be Ca-binding sites, and the amino acids at positions 196, 223, and 284 are presumed to be active centers. Therefore, it is particularly desirable not to introduce substitution or deletion at these sites.

In the case that an amino acid substitution is introduced into the amino acid sequence of the polypeptide described in (1-2) and (1-3), examples of the aspect of the amino acid substitution include conservative substitutions. That is, in the polypeptide described in (1-2) and (1-3), the amino acid substitution introduced into the amino acid sequence shown in SEQ ID NO: 1 is, for example, a substitution of the non-polar amino acid with another non-polar amino acid if the amino acid before substitution is a non-polar amino acid. If the amino acid before substitution is a non-charged amino acid, the substitution is a substitution with another non-charged amino acid. If the amino acid before substitution is an acidic amino acid, the substitution is a substitution with another acidic amino acid, and If the amino acid before substitution is a basic amino acid, the substitution is a substitution with another basic amino acid.

The phrase concerning the polypeptide described in (1-2) and (1-3), "having maltotriose-producing ability", means that the polypeptide has activity so as to exert a function as a maltotriose-producing amylase. Specifically, the phrase means that the peak detection of maltotriose can be confirmed by "<Method of Measuring Activity of Maltotriose-Producing Amylase>" described below. The phrase preferably means that the maltotriose-producing ability observed for a starchy material is equal to or higher than the maltotriose-producing ability of the polypeptide described in (1-1) (for example, the peak intensity ratio of the maltotriose detected by "<Method of Measuring Activity of Maltotriose-Producing Amylase>" is 70% or more of that in the case of the polypeptide described in (1-1)).

The maltotriose-producing amylase including the polypeptide described in (1-1) in the first embodiment has a molecular weight of about 55 kDa.

The second embodiment of the maltotriose-producing amylase of the present invention is a polypeptide selected from the group consisting of:

(2-1) a polypeptide having an amino acid sequence shown in SEQ ID NO: 2;
(2-2) polypeptides that have an amino acid sequence with substitution, addition, insertion, or deletion of one or more amino acids in the amino acid sequence shown in SEQ ID NO: 2, and have maltotriose-producing ability; and
(2-3) polypeptides that have a sequence identity to the amino acid sequence shown in SEQ ID NO: 2 of 70% or more, and have maltotriose-producing ability.

The polypeptide described in (2-1) includes the polypeptide described in (1-1) in the first embodiment, and further has a carbohydrate binding module (CBM region) on the C-terminal side. Positions 1 to 533 of the amino acid sequence shown in SEQ ID NO: 2 correspond to the polypeptide described in (1-1). Positions 582 to 674 of the amino acid sequence shown in SEQ ID NO: 2 are considered to correspond to the CBM region. It is considered that in the CBM region, a maltotriose-producing amylase is localized on the surface of the substrate (starchy material) for efficient hydrolysis. The CBM region may be directly or indirectly bonded to the C-terminal side of the polypeptide selected from the group consisting of (1-1) to (1-3) in the first embodiment. The term "being indirectly bonded" means binding through another amino acid sequence such as a linker sequence including, for example, 1 to 250, preferably 10 to 220 amino acids.

The polypeptide described in (2-2) may have an amino acid sequence into which only one modification of substitution, addition, insertion, or deletion (for example, substitution) is introduced, or may have an amino acid sequence into which two or more modifications (for example, substitution and insertion) are introduced. In the region corresponding to the CBM region of the polypeptide described in (2-2), the number of amino acids substituted, added, inserted, or deleted is to be one, two or more, or several. For example, the number is 1 to 10, preferably 1 to 8, 1 to 6, 1 to 5, or 1 to 4, more preferably 1 to 3, and particularly preferably 1 or 2, or 1.

The polypeptide described in (2-3) is to have a sequence identity to the amino acid sequence shown in SEQ ID NO: 2 of 70% or more, and the sequence identity is preferably 80% or more, 85% or more, or 90% or more, and more preferably 95% or more, 97% or more, or 98% or more, and particularly preferably 99% or more.

Here, in the polypeptide described in (2-3), the sequence identity to the amino acid sequence shown in SEQ ID NO: 2 is calculated by comparison with the amino acid sequence shown in SEQ ID NO: 2, and the term "sequence identity" is defined in the same manner as the sequence identity described in (1-3) in the first embodiment.

In the polypeptide described in (2-2) and (2-3), the positions of the amino acid in SEQ ID NO: 2, at which it is desirable not to introduce substitution or deletion, are as described above as the positions of the amino acid in SEQ ID NO: 1, at which it is desirable not to introduce substitution or deletion into the amino acid sequence of the polypeptide described in (1-2) and (1-3) in the first embodiment. Specifically, the amino acids at positions 196, 223, 284, 107, 166, 118, 119, 122, 123, 158, 161, 214, 215, 216, 217, 247, 250, 252, 253, 278, 283, 336, and 342 of the amino acid sequence shown in SEQ ID NO: 2 are considered to contribute to the activity, therefore, it is desirable not to introduce substitution or deletion at these sites. The amino acids at positions 107 and 166 are presumed to be Ca-binding sites, and the amino acids at positions 196, 223, and 284 are presumed to be active centers. Therefore, it is particularly desirable not to introduce substitution or deletion at these sites. In the CBM region, the amino acids at positions 608, 639, 651, 652, and 656 are considered to contribute to the binding to starch, therefore, if a maltotriose-producing amylase is desired to be localized on the surface of the substrate (starchy material), it is desirable not to introduce substitution or deletion at these sites.

In the case that an amino acid substitution is introduced into the amino acid sequence of the polypeptide described in (2-2) and (2-3), examples of the aspect of the amino acid substitution include conservative substitutions. The conservative substitution is defined in the same manner as the conservative substitution as the aspect of the amino acid substitution in the case that an amino acid substitution is introduced into the amino acid sequence of the polypeptide described in (1-2) and (1-3) in the first embodiment.

The phrase concerning the polypeptide described in (2-2) and (2-3), "having maltotriose-producing ability", means that the polypeptide has activity so as to exert a function as a maltotriose-producing amylase. Specifically, the phrase means that the peak detection of maltotriose can be confirmed by "<Method of Measuring Activity of Maltotriose-Producing Amylase>" described below. The phrase preferably means that the maltotriose-producing ability observed for a starchy material is equal to or higher than the maltotriose-producing ability of the polypeptide described in (2-1) (for example, the peak intensity ratio of the maltotriose detected by "<Method of Measuring Activity of Maltotriose-Producing Amylase>" is 70% or more of that in the case of the polypeptide described in (2-1)).

The maltotriose-producing amylase including the polypeptide described in (2-1) in the second embodiment has a molecular weight of about 70 kDa.

The third embodiment of the maltotriose-producing amylase of the present invention is a polypeptide selected from the group consisting of:

(3-1) a polypeptide having an amino acid sequence shown in SEQ ID NO: 3;
(3-2) polypeptides that have an amino acid sequence with substitution, addition, insertion, or deletion of one or more amino acids in the amino acid sequence shown in SEQ ID NO: 3, and have maltotriose-producing ability; and
(3-3) polypeptides that have a sequence identity to the amino acid sequence shown in SEQ ID NO: 3 of 70% or more, and have maltotriose-producing ability.

The polypeptide described in (3-1) also includes the polypeptide described in (1-1) in the first embodiment. More specifically, the polypeptide described in (3-1) includes the polypeptide described in (2-1) in the second embodiment, and further has a pro-sequence on the N-terminal. Positions 54 to 586 of SEQ ID NO: 3 correspond to the polypeptide described in (1-1) in the first embodiment, and positions 54 to 728 correspond to the polypeptide described in (2-1) in the second embodiment.

The polypeptide described in (3-2) may have an amino acid sequence into which only one modification of substitution, addition, insertion, or deletion (for example, substitution) is introduced, or may have an amino acid sequence into which two or more modifications (for example, substitution and insertion) are introduced. In the polypeptide described in (3-2), the number of amino acids substituted, added, inserted, or deleted is to be one, two or more, or several. For example, the number is 1 to 10, preferably 1 to 8, 1 to 6, 1 to 5, or 1 to 4, more preferably 1 to 3, and particularly preferably 1 or 2, or 1.

The polypeptide described in (3-3) is to have a sequence identity to the amino acid sequence shown in SEQ ID NO: 3 of 70% or more, and the sequence identity is preferably 80% or more, 85% or more, or 90% or more, and more preferably 95% or more, 97% or more, or 98% or more, and particularly preferably 99% or more.

Here, in the polypeptide described in (3-3), the sequence identity to the amino acid sequence shown in SEQ ID NO: 3 is calculated by comparison with the amino acid sequence shown in SEQ ID NO: 3, and the term "sequence identity" is defined in the same manner as the sequence identity described in (1-3) in the first embodiment.

In the case that an amino acid substitution is introduced into the amino acid sequence of the polypeptide described in (3-2) and (3-3), examples of the aspect of the amino acid substitution include conservative substitutions. The conservative substitution is defined in the same manner as the conservative substitution as the aspect of the amino acid substitution in the case that an amino acid substitution is introduced into the amino acid sequence of the polypeptide described in (1-2) and (1-3) in the first embodiment.

In the polypeptide described in (3-2) and (3-3), the positions of the amino acid in SEQ ID NO: 3, at which it is desirable not to introduce substitution or deletion, are as described above as the positions of the amino acid in SEQ ID NO: 1, at which it is desirable not to introduce substitution or deletion into the amino acid sequence of the polypeptide described in (1-2) and (1-3) in the first embodiment.

The phrase concerning the polypeptide described in (3-2) and (3-3), "having maltotriose-producing ability", means that the polypeptide has activity so as to exert a function as a maltotriose-producing amylase. Specifically, the phrase means that the peak detection of maltotriose can be confirmed by "<Method of Measuring Activity of Maltotriose-Producing Amylase>" described below. The phrase preferably means that the maltotriose-producing ability observed for a starchy material is equal to or higher than the maltotriose-producing ability of the polypeptide described in (3-1) (for example, the peak intensity ratio of the maltotriose detected by "<Method of Measuring Activity of Maltotriose-Producing Amylase>" is 70% or more of that in the case of the polypeptide described in (3-1)).

The maltotriose-producing amylase including the polypeptide described in (3-1) in the third embodiment has a molecular weight of about 77 kDa.

The enzyme activity of the maltotriose-producing amylase of the present invention can be measured by the method shown below.

### <Method of Measuring Activity of Maltotriose-Producing Amylase>

A reaction solution obtained by mixing 200 µL of a substrate solution (1% (w/v) soluble starch solution) and 50 µL of an enzyme solution is reacted by shaking at 50°C for 48 hours at 1,000 rpm, and then boiled at 100°C for 5 minutes to stop the reaction. The reaction solution after stopping the reaction is diluted 30 times with purified water and filtered through a 0.45 µm filter to obtain a sample for analysis.
The sample is analyzed by HLPC to detect the peak of maltotriose, thus measuring the activity of the maltotriose-producing amylase.

### 2. DNA

The DNA of the present invention encodes the maltotriose-producing amylase. The DNA of the present invention can be obtained by, for example, obtaining the region encoding at least one of the polypeptides described in (1-1) to (1-3) by polymerase chain reaction (PCR) or the like using, as a template, the DNA encoding any one of the polypeptides described in (3-1) to (3-3) derived from a bacterium belonging to the genus Cellulosimicrobium. The DNA encoding the maltotriose-producing amylase of the present invention can also be artificially synthesized by a gene synthesis method.

For introduction of a specific mutation into a base sequence at a specific site, a publicly known mutation-introducing method is used, and examples of the method include site-directed mutagenesis of a DNA. Specific examples of the method of converting a base in a DNA include a method in which a commercially available kit is used.

When a mutation is introduced into a base sequence of a DNA, the base sequence can be confirmed using a DNA sequencer. Once the base sequence has been determined, then a DNA encoding the maltotriose-producing amylase can be obtained by chemical synthesis, PCR employing a cloned probe as a template, or hybridization employing a DNA fragment having the base sequence as a probe.

Furthermore, a mutant, having the same function as before the mutation, of the DNA encoding the maltotriose-producing amylase can be synthesized by a method such as site-directed mutagenesis. Note that a mutation can be introduced into the DNA encoding the maltotriose-producing amylase by a publicly known method such as the Kunkel method, a gapped duplex method, or a megaprimer PCR method.

Those skilled in the art can appropriately design the base sequence of the DNA encoding the maltotriose-producing amylase of the present invention according to the amino acid sequence of the maltotriose-producing amylase of the present invention.

The first embodiment of the DNA of the present invention is a DNA encoding the polypeptide described in (1-1) to (1-3). Examples of the DNA encoding the polypeptide described in (1-1) include the DNA having the base sequence shown in SEQ ID NO: 4.

The first embodiment of the DNA of the present invention includes a DNA that encodes the polypeptide described in (1-1) to (1-3) and hybridizes under a stringent condition to a DNA including a complementary base sequence with the DNA having the base sequence shown in SEQ ID NO: 4.

The first embodiment of the DNA of the present invention further includes a DNA that encodes the polypeptide described in (1-1) to (1-3) and has a homology with the DNA having the base sequence shown in SEQ ID NO: 4 of 70% or more. The homology is preferably 80% or more or 90% or more, more preferably 95% or more, 97% or more, or 98% or more, and particularly preferably 99% or more.

The second embodiment of the DNA of the present invention is a DNA encoding any one of the polypeptides described in (2-1) to (2-3). Examples of the DNA encoding the polypeptide described in (2-1) include the DNA having the base sequence shown in SEQ ID NO: 5.

The second embodiment of the DNA of the present invention includes a DNA that encodes the polypeptide described in (2-1) to (2-3) and hybridizes under a stringent condition to a DNA including a complementary base sequence with the DNA having the base sequence shown in SEQ ID NO: 5.

The second embodiment of the DNA of the present invention further includes a DNA that encodes the polypeptide described in (2-1) to (2-3) and has a homology with the DNA having the base sequence shown in SEQ ID NO: 5 of 70% or more. The homology is preferably 80% or more or 90% or more, more preferably 95% or more, 97% or more, or 98% or more, and particularly preferably 99% or more.

The third embodiment of the DNA of the present invention is a DNA encoding any one of the polypeptides described in (3-1) to (3-3). Examples of the DNA encoding the polypeptide described in (3-1) include the DNA having the base sequence shown in SEQ ID NO: 6.

The third embodiment of the DNA of the present invention includes a DNA that encodes the polypeptide described in (3-1) to (3-3) and hybridizes under a stringent condition to a DNA including a complementary base sequence with the DNA having the base sequence shown in SEQ ID NO: 6.

The third embodiment of the DNA of the present invention further includes a DNA that encodes the polypeptide described in (3-1) to (3-3) and has a homology with the DNA having the base sequence shown in SEQ ID NO: 6 of 70% or more. The homology is preferably 80% or more or 90% or more, more preferably 95% or more, 97% or more, or 98% or more, and particularly preferably 99 or more.

Here, the term "stringent condition" refers to the condition of incubation at 50°C to 65°C for 4 hours to overnight in 6 × standard saline citrate (SSC) (1 × SSC is a solution of 0.15 M NaCl and 0.015 M sodium citrate, pH 7.0) containing 0.5% SDS, 5 × Denhardt [Denhartz's, solution of 0.1% bovine serum albumin (BSA), 0.1% polyvinylpyrrolidone, and 0.1% Ficoll 400], and 100 µg/mL salmon sperm DNA.

Hybridization under the stringent condition is specifically performed by the following method. A nylon membrane on which a DNA library or cDNA library is immobilized is prepared, and the nylon membrane is subjected to blocking at 65°C in a pre-hybridization solution containing 6 × SSC, 0.5% SDS, 5 × Denhardt, and 100 µg/mL salmon sperm DNA. Then each probe labeled with ³²P is added, followed by incubation overnight at 65°C. The nylon film is washed in 6 × SSC at room temperature for 10 minutes, in 2 × SSC containing 0.1% SDS at room temperature for 10 minutes, and in 0.2 × SSC containing 0.1% SDS at 45°C for 30 minutes, and then subjected to autoradiography to detect the DNA hybridizing to the probe specifically.

The term "homology" of a DNA refers to the value of the identity, obtained by bl2seq program (Tatiana A. Tatsusova, Thomas L. Madden, FEMS Microbiol. Lett., Vol. 174, 247-250, 1999) in BLAST PACKAGE [sgi32 bit edition, Version 2.0.12; available from the National Center for Biotechnology Information (NCBI)]. The parameters are to be set to Gap insertion Cost value: 11 and Gap extension Cost value: 1.

In the DNA of the present invention, the frequency of codon usage is preferably optimized for the host. For example, in the case of using Escherichia coli as a host, a DNA is suitable in which frequency of codon usage is optimized for Escherichia coli.

### 3. Recombinant Vector

The recombinant vector of the present invention includes a DNA encoding the maltotriose-producing amylase of the present invention. The recombinant vector of the present invention can be obtained by inserting the DNA of the present invention into an expression vector.

The recombinant vector of the present invention includes a regulator such as a promoter operatively linked to the DNA of the present invention. Examples of the regulator typically include a promoter, and if necessary, may include transcriptional elements such as an enhancer, a CCAAT box, a TATA box, and a SPI site. The phrase "being operatively linked" means that various regulators such as a promoter and an enhancer that regulate the DNA of the present invention are linked to the DNA of the present invention in an operative state in a host cell.

The expression vector is preferably constructed for gene recombination from a phage, plasmid, or virus capable of autonomously replicating in a host. Such an expression vector is publicly known, and examples of the commercially available expression vector include a pQE vector (QIAGEN), pDR540, pRIT2T (GE Healthcare Bio Sciences K. K.), and a pET vector (Merck KGaA). The expression vector is to be used in appropriate combination with a selected host cell. Preferable examples of the combination in the case of using Escherichia coli as a host cell include a combination of a pET vector and a BL21 (DE3) Escherichia coli strain and a combination of a pDR540 vector and a JM109 Escherichia coli strain.

### 4. Transformant

The transformant of the present invention is obtained by transforming a host with the DNA of the present invention or the recombinant vector of the present invention.

The host used for producing the transformant is not particularly limited as long as a gene can be introduced into the host, the host can autonomously replicate, and a character of the gene of the present invention can be expressed. Preferable examples of the host include bacteria belonging to the genus Escherichia such as Escherichia coli, bacteria belonging to the genus Bacillus such as Bacillus subtilis, and bacteria belonging to the genus Pseudomonas such as Pseudomonas putida; Actinobacteria; yeast; and filamentous fungi. In addition, examples of the host may include animal cells, insect cells, and plants. Among these hosts, Escherichia coli is particularly preferable. The host may be a bacterium belonging to the genus Cellulosimicrobium from which the maltotriose-producing amylase of the present invention is derived.

The transformant of the present invention can be obtained by introducing the DNA of the present invention or the recombinant vector of the present invention into a host. The method of introducing the DNA of the present invention or the recombinant vector of the present invention is not particularly limited as long as the gene of interest can be introduced into a host. Where the DNA is introduced is not particularly limited as long as the gene of interest can be expressed, and the DNA may be introduced into a plasmid or a genome. Specific examples of the method of introducing the DNA of the present invention or the recombinant vector of the present invention include a recombinant vector method and a genome editing method.

The condition for introducing the DNA of the present invention or the recombinant vector of the present invention into a host is to be appropriately set according to the method of the introduction, the kind of host, and the like. In the case of using a bacterium as a host, examples of the method include a method in which a competent cell by calcium ion treatment is used, and an electroporation method. In the case of using yeast as a host, examples of the method include an electroporation method, a spheroplast method, and a lithium acetate method. In the case of using an animal cell as a host, examples of the method include an electroporation method, a calcium phosphate method, and a lipofection method. In the case of using an insect cell as a host, examples of the method include a calcium phosphate method, a lipofection method, and an electroporation method. In the case of using a plant as a host, examples of the method include an electroporation method, an Agrobacterium method, a particle gun method, and a PEG method.

### 5. Method for Producing Maltotriose-Producing Amylase

The maltotriose-producing amylase of the present invention can be produced by culturing the transformant of the present invention. The maltotriose-producing amylase of the present invention can also be produced by culturing a producing bacterium, Cellulosimicrobacterium sp. (untransformed). In the case of culturing the producing bacterium, Cellulosimicrobacterium sp. (untransformed), the maltotriose-producing amylase of the present invention is produced in a state of mixing any one of the polypeptides described in (1-1) to (1-3) (the polypeptide in the first embodiment), any one of the polypeptides described in (2-1) to (2-3) (the polypeptide in the second embodiment), and any one of the polypeptides described in (3-1) to (3-3) (the polypeptide in the third embodiment) through processing. Meanwhile, in the case of using the transformant of the present invention, only a gene encoding any one of the polypeptides in the first embodiment, the second embodiment, and the third embodiment can be expressed in a host according to the DNA introduced. This fact makes it possible both to produce the maltotriose-producing amylase including any one of the polypeptides in the first embodiment, the second embodiment, and the third embodiment singly, and to produce the maltotriose-producing amylase in a state of mixing the polypeptide in the first embodiment, the polypeptide in the second embodiment, and the polypeptide in the third embodiment.

The culture condition of the transformant of the present invention is to be appropriately set in consideration of the nutritional and physiological properties of the host, and liquid culture is preferable. In the case of industrial production, aeration-agitation culture is preferable.

The transformant of the present invention is cultured, and the culture supernatant or the bacterial cell is collected by a method such as centrifugation of the culture solution. In the case that the maltotriose-producing amylase of the present invention is accumulated in the bacterial cell, the cell is treated by a mechanical method employing an ultrasonic wave, French press, or the like, or by a lytic enzyme such as lysozyme, and if necessary, by using an enzyme such as a protease and a surfactant such as sodium dodecyl sulfate (SDS) for solubilization to obtain a water-soluble fraction containing the maltotriose-producing amylase of the present invention.

Furthermore, selection of an appropriate expression vector and host also allows secretion of the expressed maltotriose-producing amylase of the present invention in the culture solution.

The culture solution or water-soluble fraction containing the maltotriose-producing amylase of the present invention obtained as described above may be directly subjected to purification treatment, or may be subjected to purification treatment after concentration of the polypeptide of the present invention in the culture solution or water-soluble fraction.

The concentration can be performed by, for example, vacuum concentration, membrane concentration, salting-out treatment, or a fractional precipitation method by use of a hydrophilic organic solvent (such as methanol, ethanol, or acetone).

The purification treatment of the maltotriose-producing amylase of the present invention can be performed by, for example, appropriately combining methods such as gel filtration, hydrophobic chromatography, ion exchange chromatography, and affinity chromatography.

The maltotriose-producing amylase of the present invention purified in this manner may be powdered by freeze-drying, vacuum-drying, spray-drying, or the like, if necessary.

### 6. Enzyme Preparation

The enzyme preparation of the present invention contains the maltotriose-producing amylase of the present invention as an active ingredient. The enzyme preparation may contain, as the maltotriose-producing amylase, only the maltotriose-producing amylase including any one of the polypeptides described in (1-1) to (1-3), may contain only the maltotriose-producing amylase including any one of the polypeptides described in (2-1) to (2-3), or may contain only the maltotriose-producing amylase including any one of the polypeptides of the maltotriose-producing amylase including the polypeptide described in (3-1) to (3-3). The enzyme may contain a plurality of the maltotriose-producing amylases. The kind and the content ratio of the contained maltotriose-producing amylase can be appropriately selected according to the substrate.

The enzyme preparation of the present invention can contain, in addition to the maltotriose-producing amylase of the present invention, an additive selected from the group consisting of excipients, buffers, suspending agents, stabilizers, preservatives, antiseptics, and physiological saline. Examples of the excipients include starch, dextrin, maltose, trehalose, lactose, D-glucose, sorbitol, D-mannitol, sucrose, and glycerol. As the buffer, phosphates, citrates, acetates, and the like can be used. Examples of the stabilizers include propylene glycol and ascorbic acid. Examples of the preservatives include sodium chloride, phenol, benzalkonium chloride, benzyl alcohol, chlorobutanol, and methylparaben. Examples of the antiseptics include sodium chloride, ethanol, benzalkonium chloride, parahydroxybenzoic acid, and chlorobutanol.

The content of the maltotriose-producing amylase in the enzyme preparation of the present invention is appropriately set within a range in which the effect of the maltotriose-producing amylase is exhibited.

The enzyme preparation of the present invention may contain another enzyme. Examples of another enzyme include amylases (a-amylases, β-amylases, glucoamylases), glucosidases (a-glucosidases, β-glucosidases), galactosidases (α-galactosidases, β-galactosidases), proteases (acidic proteases, neutral proteases, alkaline proteases), peptidases (leucine peptidases, aminopeptidases), lipases, esterases, cellulases, phosphatases (acidic phosphatases, alkaline phosphatases), nucleases, deaminases, oxidases, dehydrogenases, glutaminases, pectinases, catalases, dextranases, transglutaminases, protein deamidation enzymes, and pullulanases.

The form of the enzyme preparation of the present invention is not particularly limited, and examples of the form include liquid, powder, and granule forms. The enzyme preparation of the present invention can be prepared by a publicly known method.

### 7. Method for Producing Maltotriose

The method for producing maltotriose of the present invention includes the step of producing maltotriose from a starchy material using the above-described maltotriose-producing amylase.

Examples of the starchy material include amylose, amylopectin, glycogen, and starch; and partial starch hydrolysates, such as amylodextrin, maltodextrin, and maltooligosaccharide, obtained by partially hydrolyzing the above-described starchy materials with an amylase or acid. Examples of the partial starch hydrolysate obtained by hydrolysis with an amylase include partial hydrolysates obtained by hydrolyzing amylose, amylopectin, glycogen, starch, or the like with an amylase such as α-amylase (EC 3. 2. 1. 1), a maltopentaose-producing amylase, or maltohexaose-producing amylase (EC 3. 2. 1. 98). Furthermore, during preparation of the partial hydrolysate, a starch debranching enzyme such as pullulanase (EC 3. 2. 1. 41) or isoamylase (EC 3. 2. 1. 68) may be allowed to act.

Examples of the starch include starches derived from corn, wheat, rice, potato, sweet potato, and tapioca. These starches can be used in the form of a liquefied starch solution obtained by gelatinization and liquefaction.

When the maltotriose-producing amylase of the present invention is allowed to act on a starchy material, the concentration of the starchy material solution is not particularly limited. From an industrial point of view, the concentration is, for example, 10% (w/v) or more. The reaction temperature is not particularly limited as long as the reaction proceeds, and is specifically up to about 65°C, and preferably 45 to 60°C. The reaction pH is usually 5 to 9, and preferably 5.5 to 7.5. The amount of the enzyme used is to be appropriately selected according to the desired enzyme reaction rate.

In this maltotriose-producing reaction, another enzyme may be simultaneously used in combination to increase the maltotriose content in the saccharified solution. For example, the maltotriose content in the saccharified solution can be increased by using a starch debranching enzyme such as a pullulanase or an isoamylase in combination.

The reaction solution obtained by the above-described reaction may be used as it is as a maltotriose-containing sugar solution, but the maltotriose-containing sugar solution is preferably further purified. As the purification method, a normal method used for purification of sugar is to be appropriately employed. Examples of the method include one or two or more purification methods such as decolorization with activated charcoal, desalting with H⁺ or OH⁻ ion exchange resin, fractionation by column chromatography such as ion exchange column chromatography, activated charcoal column chromatography, or silica gel column chromatography, separation with an organic solvent such as an alcohol or acetone, and separation with a membrane having suitable separating function.

Examples of the method of obtaining a high-purity maltotriose-containing saccharide include ion exchange column chromatography. Specifically, an impurity saccharide is removed by column chromatography using a strong acid cation exchange resin to produce maltotriose having an improved content of the objective or to produce a saccharide containing such maltotriose.

The maltotriose-containing saccharide obtained in this manner or a saccharide having an improved content of the maltotriose-containing saccharide can be concentrated into a syrup-like product. The syrup-like product can also be dried and powdered into a powder-like product.

The maltotriose obtained by the method for producing of the present invention or a saccharide containing the maltotriose can be used as an additive for various compositions such as foods and drinks, favorite foods, animal foods, feeds, cosmetics, quasi-drugs, and pharmaceuticals in the form of syrup or powder as a sweetener, a taste improver, a quality improver, a stabilizer, or the like.

### EXAMPLES

Hereinafter, the present invention will be specifically described with reference to examples, but the present invention should not be construed as being limited to the following examples.

As described below, an α-amylase was obtained from a candidate for a producing bacterium, and the maltotriose-producing activity of the obtained α-amylase was confirmed. Furthermore, the candidate was identified for a producing bacterium that produced the α-amylase whose maltotriose-producing activity was confirmed. In addition, amino acid sequence analysis and base sequence determination were performed on the α-amylase whose maltotriose-producing activity was confirmed.

### [Test Example 1: Acquisition of α-Amylase from Candidate for Producing Bacterium]

### [1] Culture of Candidate for Producing Bacterium

A liquid medium having the following composition was put in an Erlenmeyer flask and sterilized at 121°C for 20 minutes using an autoclave. A candidate for a producing bacterium was inoculated into the liquid medium and cultured at 30°C for 3 days.

**[Table 1]**

| ○ Composition of medium | |
|---|---|
| Component | Final concentration % (w/v) |
| Corn steep liquor | 10.0% |
| Soluble starch | 2.0% |
| K₂HPO₄ | 0.2% |
| MgSO₄•7H₂O | 0.1% |
| CaCl₂ | 0.01% |
| NaNO₃ | 0.1% |
| Tween 40 | 0.1% |
| pH 7.0 ± 0.1 | 121°C, 20 min. sterilized |

**[Table 2]**

| ○ Culture conditions | |
|---|---|
| Item | Set value |
| Volume | 40 mL/200 mL Erlenmeyer flask |
| Amount of inoculation | One platinum loop |
| Temperature | 30°C |
| Culture time | About 72 h |
| Shaking speed | 150 rpm |

### [2] Purification of Enzyme

The enzyme produced by the candidate for a producing bacterium was purified through bacterial cell separation, ultrafiltration, ammonium sulfate fractionation, dialysis, first-time treatment with DEAE-Sepharose, second-time treatment with DEAE-Sepharose, first-time treatment with Sephadex G-150, and second-time treatment with Sephadex G-150.

The bacterial cell separation was performed by centrifuging the culture supernatant (7,000 rpm, 5 min). The ultrafiltration was performed by using an ultrafiltration membrane (AIV membrane, Asahi Kasei Corp.) for the centrifuged supernatant. Through the ammonium sulfate fractionation, 0 to 40 fractions were obtained. The dialysis was performed using a Tris-hydrochloric acid buffer (pH 7.0). In the first-time treatment with DEAE-Sepharose, a DEAE-Sepharose column was used. The column was equilibrated and washed with a 2.5 × 10⁻³ M Tris-hydrochloric acid buffer (pH 7.0) containing 10⁻³ M L-cysteine for elution by KCl (0 to 1.0 M gradient) to collect amylase fractions. The amylase fractions were concentrated with a membrane filter, and then fractionated again with DEAE-Sepharose. In the second-time treatment with DEAE-Sepharose, the operation was performed in the same manner as in the first-time treatment with DEAE-Sepharose. In the first-time treatment with Sephadex G-150, a Sephadex G-150 column was used. The column was equilibrated and washed with a 2.5 × 10⁻³ M Tris-hydrochloric acid buffer (pH 7.0) containing 10⁻³ M L-cysteine for elution by KCl (0 to 1.0 M gradient) to collect amylase fractions. The amylase fractions were concentrated with a membrane filter, and then fractionated again with a Sephadex G-150 column. In the second-time Sephadex G-150, the operation was performed in the same manner as in the first-time Sephadex G-150.

The term "amylase fraction" refers to a fraction in which the α-amylase activity is confirmed using an α-amylase measurement kit (manufactured by Kikkoman Biochemifa Company) in accordance with the protocol of the kit.

The obtained fraction was developed by SDS-PAGE. In the SDS-PAGE, 10 µL of the obtained fraction was diluted with 5 µL of an SDS sample buffer [Tris-HCL buffer (pH 6.8) 0.125 M, SDS 4% (w/v), sucrose 10% (w/v), BPB (bromophenol blue) 0.01% (w/v), DTT (dithiothreitol) 0.2 M] to prepare an electrophoretic sample, the electrophoretic sample was boiled at 99.9°C for 10 minutes, and then 10 µL of the electrophoretic sample was subjected to an electrophoretic gel [SDS gel: SuperSep (trademark) Ace 15% (manufactured by FUJIFILM Wako Pure Chemical Corporation)] to perform electrophoresis. The electrophoretic gel was transferred to a poly vinylidene di-fluoride (PVDF) membrane [Trans-Blot Turbo (trademark) Mini PVDF Transfer Pack (manufactured by Bio-Rad Laboratories, Inc.)], and the transfer membrane was stained with a stain solution [50% (v/v) methanol aqueous solution containing 0.1% (w/v) of CBB R-250] and decolorized with a 50% (v/v) methanol aqueous solution.

Fig. 1 shows the result of staining the SDS gel. The rightmost lane in Fig. 1 shows a molecular marker. Fig. 1 suggests that the obtained enzyme takes three forms through processing. The enzyme indicated by arrow 1 had a molecular weight of 55 kDa, the enzyme indicated by arrow 2 had a molecular weight of 70 kDa, and the enzyme indicated by arrow 3 had a molecular weight of 77 kDa. It was confirmed that the enzyme having a molecular weight of 55 kDa indicated by arrow 1 was purified as a single substance. The α-amylase activity of the 55 kDa purified fraction in Fig. 1 was confirmed by the above-described α-amylase measurement kit.

### [Test Example 2: Confirmation of Maltotriose-Producing Activity]

Furthermore, the maltotriose-producing activity of the 55 kDa purified fraction in Fig. 1 was confirmed as follows.

### [1] Preparation of Substrate Solution

In about 60 mL of purified water, 1 g of soluble starch was suspended, and the resulting suspension was heated at 100°C for 5 minutes to dissolve the soluble starch. After cooling, the resulting solution was diluted with purified water to 100 mL to obtain a substrate solution containing soluble starch at a content of 1% (w/v).

### [2] Preparation of Reaction Solution

For preparation of a reaction solution, 200 µL of the substrate solution and 50 µL of the enzyme solution to be analyzed were mixed to prepare 250 µL of a reaction solution.

### [3] Preparation of Sample for Analysis

The reaction solution was reacted by shaking at 50°C for 48 hours at 1,000 rpm, and then boiled at 100°C for 5 minutes to stop the reaction. The reaction solution after stopping the reaction was diluted 30 times with purified water and filtered through a 0.45 µm filter to obtain a filtrate as a sample for analysis.

The sample was put in a sample vial for analysis and subjected to HPLC analysis under the following conditions.

**[Table 3]**

| ○ HPLC analysis | |
|---|---|
| Device: | Ultra high performance liquid chromatograph Nexera (SHIMADZU CORPORATION) |
| Column: | CK04S 10 10 mmcp × 200 mm |
| Oven: | 80°C |
| Mobile phase: | Purified water |
| Flow rate: | 0.4 mL/min |
| Injection volume: | 3 µL |
| Analysis time: | 30 min |
| Detector: | ELS |
| Detecting temperature: | 60°C |

As a result of HPLC analysis, a peak of maltotriose as a product was detected. That is, the maltotriose-producing activity of the 55 kDa purified fraction was confirmed.

In the same manner, the maltotriose-producing activity was confirmed for the 70 kDa purified fraction and the 77 kDa purified fraction in Fig. 1.

### [Test Example 3: Identification of Producing Bacterium of α-Amylase Having Maltotriose-Producing Activity]

The candidate for a producing bacterium of the α-amylase whose maltotriose-producing activity was confirmed was subjected to 16S rRNA analysis. As a result, the candidate for a producing bacterium was identified as Cellulosimicrobacterium sp.

### [Test Example 4: Amino Acid Sequence Analysis and Base Sequence Determination of α-Amylase Having Maltotriose-Producing Activity]

### [1] Analysis of N-Terminal Amino Acid Sequence and Internal Amino Acid Sequence

Three bands (55 kDa, 70 kDa, 77 kDa) obtained in the item [2] of Test Example 1 were cut out and analyzed with a protein sequencer. As a result, the N-terminal amino acid sequence of each band was identified. Furthermore, each band was treated with trypsin and then analyzed by LC-MSMS to identify the internal amino acid sequence.

### [2] Determination of Base Sequence

### [2-1]PCR

Primers were designed on the basis of the identified N-terminal amino acid sequence and internal amino acid sequence. PCR amplification was performed using 20 µL/tube of a PCR reaction solution. The composition of the PCR reaction solution and the PCR conditions are as follows. The PCR product was run on a 1% agarose gel, and single amplification was confirmed.

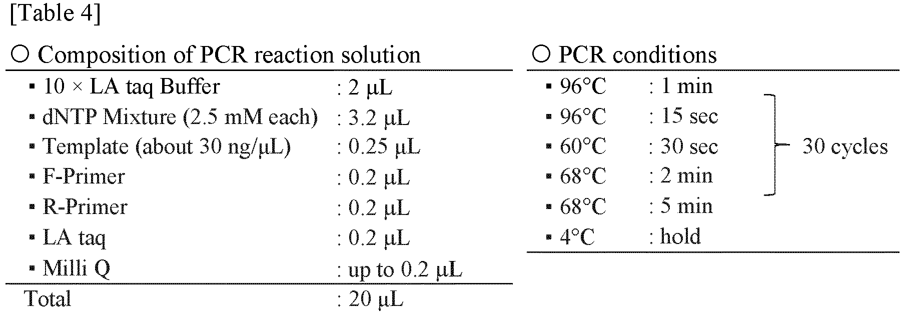

### [2-2] TA Cloning

A ligation reaction was carried out at 16°C for 30 minutes using a ligation reaction solution in which 2 µL of the PCR product, 1 µL of T-Vecor pMD20, and 3 µL of Ligation Mix were mixed, and then the resulting ligation mixture was transformed into 25 µL of E. coli BL21 (DE3). About 30 µL of the obtained transformation solution was applied to a liquid medium containing LB (Thermo Fisher Scientific K. K.) and Amp (final concentration 100 µg/mL) (hereinafter referred to as "LB + Amp liquid medium") plate, and cultured at 37°C, O/N.

### [2-3] Extraction of Plasmid

The transformant was cultured in an LB + Amp liquid medium (2 mL) at 37°C, O/N. After the culture was completed, the cell was collected and the plasmid was extracted by the miniprep method.

### [2-4] Sequence Analysis

Sequence analysis was performed by the Sanger method to determine the partial base sequence.

### [2-5] Full-Length Base Sequence Analysis

Colony hybridization was performed using a probe prepared on the basis of the obtained partial base sequence, and the gene sequence of the obtained positive clone was analyzed to determine the target base sequence. As a result, from the 55 kDa band, the base sequence of SEQ ID NO: 4 was obtained. From the 70 kDa band, the base sequence of SEQ ID NO: 5 was obtained. From the 77 kDa band, the base sequence of SEQ ID NO: 6 was obtained.

### [3] Determination of Amino Acid Sequence

From the above-described base sequence, the amino acid sequence was determined. The amino acid sequence encoded by the base sequence of SEQ ID NO: 4 was SEQ ID NO: 1, the amino acid sequence encoded by the base sequence of SEQ ID NO: 5 was SEQ ID NO: 2, and the amino acid sequence encoded by the base sequence of SEQ ID NO: 6 was SEQ ID NO: 3. That is, it was found that Test Example 1 gave the maltotriose-producing amylases having amino acid sequences of SEQ ID NOs: 1, 2, and 3.

### [Test Example 5: Recombinant Production of Maltotriose-Producing Amylase]

### [1] Construction of Expression Vector

### [1-1] PCR

In order to amplify the amylase gene, the primer shown in SEQ ID NO: 7 was designed as a forward primer, and the primer shown in SEQ ID NO: 8 was designed as a reverse primer. PCR amplification was performed using 50 µL/tube of a PCR reaction solution. The composition of the PCR reaction solution and the PCR conditions are as follows. The PCR product was run on a 1% agarose gel, and single amplification was confirmed.

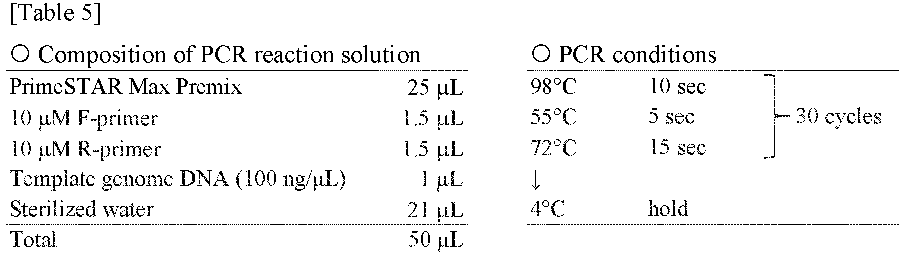

### [1-2. Ligation and Transformation]

A ligation reaction was carried out at 16°C for 30 minutes using a ligation reaction solution (5 µL) in which the PCR product and pUBCM21 (a shuttle vector of pUC19 and pUB110) were mixed, and then the resulting ligation mixture was transformed into 50 µL of E. coli DH5α. About 55 µL of the obtained transformation solution was applied to an LB + Amp (100 µg/mL) plate and cultured at 37°C, O/N. The composition of the ligation reaction solution is as follows.

**[Table 6]**

| ○ Composition of ligation reaction solution | |
|---|---|
| PCR product | 2 µL |
| pUBCM21 | 1 µL |
| Ligatin Mix | 3 µL |
| Total | 6 µL |

### [1-3. Extraction of Plasmid]

The plasmid was extracted by the same method as in 2-3 in Test Example 4 to obtain a plasmid, pUBCM21-amy.

### [2] Transformation of Bacillus subtilis

Using the obtained vector pUBCM21-amy, amyE-deficient strain derived from Bacillus subtilis 168 strain was transformed. The transformation was performed by a method following the protocol of B. subtilis Secretory Protein Expression System (manufactured by Takara Bio Inc.).

### [3] Production of Maltotriose-Producing Amylase

### [3-1] Culture of Transformed Strain

The transformed strain was inoculated into a liquid medium containing LB (Thermo Fisher Scientific K. K.) and kanamycin (20 µg/mL), and cultured with shaking at 37°C for 4 days. After the start of culture, 0.5 mL of the culture solution was sampled every day and centrifuged at 4°C at 15,000 rpm for 5 minutes to collect a supernatant, thus obtaining a maltotriose-producing amylase.

### [3-2] Confirmation of α-Amylase Productivity

The activity of the collected supernatant (maltotriose-producing amylase fraction) was measured, and α-amylase productivity was confirmed by SDS-PAGE. The activity was measured using an α-amylase measurement kit (Kikkoman Biochemifa Company) in accordance with the protocol of the kit. The SDS-PAGE was performed in the same manner as the SDS-PAGE in Test Example 1.

As a result of the activity measurement, significant activity was confirmed in the maltotriose-producing amylase fraction as compared with the negative control, pUBCM21 (empty vector-introduced strain). As a result of the SDS-PAGE, three bands derived from the α-amylases were confirmed by optimizing codons for Bacillus subtilis. These three bands were presumed, from their molecular weights, to be the bands of the amylases having amino acid sequences of SEQ ID NOs: 1, 2, and 3. That is, it is considered that Test Example 5 gave the maltotriose-producing amylases having amino acid sequences of SEQ ID NOs: 1, 2, and 3.

SEQ ID NOs: 7 and 8 shows primers.

## Claims

1. A maltotriose-producing amylase comprising a polypeptide selected from the group consisting of:
(1-1) a polypeptide having an amino acid sequence shown in SEQ ID NO: 1;
(1-2) polypeptides that have an amino acid sequence with substitution, addition, insertion, or deletion of one or more amino acids in the amino acid sequence shown in SEQ ID NO: 1, and have maltotriose-producing ability;
(1-3) polypeptides that have a sequence identity to the amino acid sequence shown in SEQ ID NO: 1 of 70% or more, and have maltotriose-producing ability;
(2-1) a polypeptide having an amino acid sequence shown in SEQ ID NO: 2;
(2-2) polypeptides that have an amino acid sequence with substitution, addition, insertion, or deletion of one or more amino acids in the amino acid sequence shown in SEQ ID NO: 2, and have maltotriose-producing ability;
(2-3) polypeptides that have a sequence identity to the amino acid sequence shown in SEQ ID NO: 2 of 70% or more, and have maltotriose-producing ability;
(3-1) a polypeptide having an amino acid sequence shown in SEQ ID NO: 3;
(3-2) polypeptides that have an amino acid sequence with substitution, addition, insertion, or deletion of one or more amino acids in the amino acid sequence shown in SEQ ID NO: 3, and have maltotriose-producing ability; and
(3-3) polypeptides that have a sequence identity to the amino acid sequence shown in SEQ ID NO: 3 of 70% or more, and have maltotriose-producing ability.

2. A DNA encoding the maltotriose-producing amylase according to claim 1.

3. A recombinant vector comprising the DNA according to claim 2.

4. A transformant obtained by transforming a host with the DNA according to claim 2 or the recombinant vector according to claim 3.

5. A method for producing the maltotriose-producing amylase according to claim 1, the method comprising the step of culturing the transformant according to claim 4.

6. An enzyme preparation comprising the maltotriose-producing amylase according to claim 1.

7. A method for producing maltotriose, the method comprising the step of producing maltotriose from a starchy material using the maltotriose-producing amylase according to claim 1.


SEQUENCE LISTING

<110>  Amano Enzyme Inc.

<120>  Maltotriose-Forming Amylase

<130>  19060WO

<150>  JP2018-204917
<151>  2018-10-31

<160>  8

<170>  PatentIn version 3.5

<210>  1
<211>  533
<212>  PRT
<213>  Cellulosimicrobacterium.sp

<400>  1

Ala Pro Ala Ala Ala Ala Pro Pro Val Pro Thr Pro Val Val Asp Ser
1               5                   10                  15

Pro Asn Gly Arg Gly Asp Val Ile Leu Asn Leu Phe Gln Trp Thr Trp
            20                  25                  30

Asp Ser Val Ala Ala Glu Cys Thr Ser Thr Ile Gly Pro Ala Gly Phe
        35                  40                  45

Gly Tyr Val Gln Val Ser Pro Pro Gln Glu His Ile Gln Gly Thr Ala
    50                  55                  60

Trp Trp Thr Ser Tyr Gln Pro Val Ser Tyr Lys Leu Glu Ser Lys Leu
65                  70                  75                  80

Gly Thr Arg Ala Glu Phe Gln Arg Met Val Ser Thr Cys Lys Ala Ala
                85                  90                  95

Gly Val Gly Val Ile Val Asp Ala Val Val Asn His Thr Ala Gly Ala
            100                 105                 110

Asp Thr Gly Ser Gly Thr Gly Thr Gly Gly Thr Ser Tyr Ser Val Asp
        115                 120                 125

Ser Phe Pro Gly Val Pro Tyr Gly Pro Asn Asp Phe Asn Asp Cys Arg
    130                 135                 140

Ser Asn Ile Ser Asn Tyr Gly Asp Arg Tyr Asn Val Gln Asn Cys Arg
145                 150                 155                 160

Leu Val Ser Leu Gln Asp Leu Arg Thr Gly Ser Asp Tyr Val Arg Asp

                165                 170                 175

Lys Ile Ala Gly Tyr Leu Asn Asp Leu Leu Ser Leu Gly Val Ser Gly
            180                 185                 190

Phe Arg Ile Asp Ala Ala Lys His Ile Pro Ala Ala Asp Leu Ala Ala
        195                 200                 205

Ile Lys Ala Arg Leu Thr Asn Pro Asp Val Phe Trp Val His Glu Val
    210                 215                 220

Ile Gly Ala Ser Gly Glu Pro Ile Gln Pro Ser Glu Tyr Leu Gly Ser
225                 230                 235                 240

Gly Asp Ser His Glu Phe Phe Tyr Ala Arg Glu Leu Lys Ser Arg Phe
                245                 250                 255

Asp Gly Gln Ile Lys Asp Leu Arg Thr Ile Gly Asp Asn Lys Leu Pro
            260                 265                 270

Ser Asp Arg Ala Gly Val Phe Val Asp Asn His Asp Thr Glu Arg Asn
        275                 280                 285

Gly Glu Thr Met Ser Tyr Lys Trp Gly Ala Lys Tyr Arg Leu Ala Asn
    290                 295                 300

Ala Phe Met Leu Ser Trp Pro Tyr Gly Ala Pro Ser Val Tyr Ser Gly
305                 310                 315                 320

Tyr Thr Trp Ser Asp Lys Asp Ala Gly Ala Pro Gly Ala Ser Ala Thr
                325                 330                 335

Ser Val Pro Asp Ala Ser Cys Ala Asp Ser Ala Trp Val Cys Thr Gln
            340                 345                 350

Arg Trp Thr Glu Ile Ala Gly Met Val Gly Phe His Asn Ala Val Ala
        355                 360                 365

Gly Thr Pro Val Thr Gly Trp Trp Asp Asp Gly Asn Asn His Ile Ala
    370                 375                 380

Tyr Gly Arg Gly Ala Lys Gly Tyr Val Thr Ile Asn Asn Ser Ala Asn
385                 390                 395                 400

Ala Val Thr Arg Thr Tyr Gln Thr Ser Leu Pro Ala Gly Thr Tyr Cys
                405                 410                 415

Asp Val Val Ala Ser Lys Asp Cys Ser Lys Thr Val Ser Val Ser Gly
            420                 425                 430

Ser Gly Thr Phe Thr Ala Thr Val Pro Ala Tyr Gly Ala Leu Ala Leu
        435                 440                 445

His Val Gly Ala Arg Gly Asp Gly Gly Thr Gly Gly Pro Gly Pro Val
    450                 455                 460

Asp Pro Ala Gly Thr Thr Thr Val Tyr Tyr Ala Thr Asp Lys Gly Trp
465                 470                 475                 480

Asn Ala Tyr Asn Val His Tyr Lys Val Gly Thr Gly Ala Trp Thr Ala
                485                 490                 495

Val Pro Gly Val Pro Met Thr Ala Ala Cys Thr Gly Trp Val Ser Arg
            500                 505                 510

Gln Ile Ser Leu Pro Ser Gly Ala Thr Gly Ala Ala Ala Thr Val Thr
        515                 520                 525

Ala Ala Phe Thr Asn
    530

<210> 2
<211> 675
<212> PRT
<213> Cellulosimicrobacterium.sp

<400> 2

Ala Pro Ala Ala Ala Ala Pro Pro Val Pro Thr Pro Val Val Asp Ser
1               5                   10                  15

Pro Asn Gly Arg Gly Asp Val Ile Leu Asn Leu Phe Gln Trp Thr Trp
            20                  25                  30

Asp Ser Val Ala Ala Glu Cys Thr Ser Thr Ile Gly Pro Ala Gly Phe
        35                  40                  45

Gly Tyr Val Gln Val Ser Pro Pro Gln Glu His Ile Gln Gly Thr Ala
    50                  55                  60

Trp Trp Thr Ser Tyr Gln Pro Val Ser Tyr Lys Leu Glu Ser Lys Leu
65                  70                  75                  80

Gly Thr Arg Ala Glu Phe Gln Arg Met Val Ser Thr Cys Lys Ala Ala
                85                  90                  95

Gly Val Gly Val Ile Val Asp Ala Val Val Asn His Thr Ala Gly Ala
        100                 105                 110

Asp Thr Gly Ser Gly Thr Gly Thr Gly Gly Thr Ser Tyr Ser Val Asp
    115                 120                 125

Ser Phe Pro Gly Val Pro Tyr Gly Pro Asn Asp Phe Asn Asp Cys Arg
    130                 135                 140

Ser Asn Ile Ser Asn Tyr Gly Asp Arg Tyr Asn Val Gln Asn Cys Arg
145                 150                 155                 160

Leu Val Ser Leu Gln Asp Leu Arg Thr Gly Ser Asp Tyr Val Arg Asp
                165                 170                 175

Lys Ile Ala Gly Tyr Leu Asn Asp Leu Leu Ser Leu Gly Val Ser Gly
            180                 185                 190

Phe Arg Ile Asp Ala Ala Lys His Ile Pro Ala Ala Asp Leu Ala Ala
        195                 200                 205

Ile Lys Ala Arg Leu Thr Asn Pro Asp Val Phe Trp Val His Glu Val
    210                 215                 220

Ile Gly Ala Ser Gly Glu Pro Ile Gln Pro Ser Glu Tyr Leu Gly Ser
225                 230                 235                 240

Gly Asp Ser His Glu Phe Phe Tyr Ala Arg Glu Leu Lys Ser Arg Phe
                245                 250                 255

Asp Gly Gln Ile Lys Asp Leu Arg Thr Ile Gly Asp Asn Lys Leu Pro
            260                 265                 270

Ser Asp Arg Ala Gly Val Phe Val Asp Asn His Asp Thr Glu Arg Asn
        275                 280                 285

Gly Glu Thr Met Ser Tyr Lys Trp Gly Ala Lys Tyr Arg Leu Ala Asn
    290                 295                 300

Ala Phe Met Leu Ser Trp Pro Tyr Gly Ala Pro Ser Val Tyr Ser Gly
305                 310                 315                 320

Tyr Thr Trp Ser Asp Lys Asp Ala Gly Ala Pro Gly Ala Ser Ala Thr
                325                 330                 335

Ser Val Pro Asp Ala Ser Cys Ala Asp Ser Ala Trp Val Cys Thr Gln
            340                 345                 350

Arg Trp Thr Glu Ile Ala Gly Met Val Gly Phe His Asn Ala Val Ala
        355                 360                 365

Gly Thr Pro Val Thr Gly Trp Trp Asp Asp Gly Asn Asn His Ile Ala
    370                 375                 380

Tyr Gly Arg Gly Ala Lys Gly Tyr Val Thr Ile Asn Asn Ser Ala Asn
385                 390                 395                 400

Ala Val Thr Arg Thr Tyr Gln Thr Ser Leu Pro Ala Gly Thr Tyr Cys
                405                 410                 415

Asp Val Val Ala Ser Lys Asp Cys Ser Lys Thr Val Ser Val Ser Gly
            420                 425                 430

Ser Gly Thr Phe Thr Ala Thr Val Pro Ala Tyr Gly Ala Leu Ala Leu
        435                 440                 445

His Val Gly Ala Arg Gly Asp Gly Gly Thr Gly Gly Pro Gly Pro Val
    450                 455                 460

Asp Pro Ala Gly Thr Thr Thr Val Tyr Tyr Ala Thr Asp Lys Gly Trp
465                 470                 475                 480

Asn Ala Tyr Asn Val His Tyr Lys Val Gly Thr Gly Ala Trp Thr Ala
                485                 490                 495

Val Pro Gly Val Pro Met Thr Ala Ala Cys Thr Gly Trp Val Ser Arg
            500                 505                 510

Gln Ile Ser Leu Pro Ser Gly Ala Thr Gly Ala Ala Ala Thr Val Thr
        515                 520                 525

Ala Ala Phe Thr Asn Gly Ser Gly Ala Trp Asp Ser Asn Gly Gly Lys
    530                 535                 540

Asp Tyr Ser Leu Ser Gly Ala Ala Val Ala Val Ser Gly Gly Thr Val
545                 550                 555                 560

Thr Ala Gly Asn Pro Cys Asp Gly Gly Gly Ser Pro Gly Gly Pro Val
                565                 570                 575

Glu Gly Gln Gly Asp Ala Ser Phe Ser Val Thr Ala Thr Thr Thr Trp
            580                 585                 590

Gly Gln Thr Val His Val Val Gly Ser Ile Pro Ala Leu Gly Ser Trp
        595                 600                 605

Ala Pro Ala Asn Gly Val Pro Leu Ser Ser Ser Ala Tyr Pro Val Trp
    610                 615                 620

Thr Ala Arg Val Asp Leu Pro Ala Gly Thr Thr Phe Gln Tyr Lys Tyr
625                 630                 635                 640

Val Lys Arg Asp Gly Gly Gly Asn Val Val Trp Glu Ser Gly Gly Asn
                645                 650                 655

Arg Thr Ala Thr Val Gly Ala Asp Gly Ser Val Thr Leu Ser Asp Thr
            660                 665                 670

Trp Arg Ser
        675

<210>  3
<211>  728
<212>  PRT
<213>  Cellulosimicrobacterium.sp

<400>  3

Met Thr Arg Thr Thr Asp Leu Ala Gly Pro Pro Ala Pro Thr Pro Val
1               5                   10                  15

Pro Ala Gly Arg Pro Ala Arg Pro Arg Gly Arg Arg Leu Arg Arg Val
            20                  25                  30

Leu Ala Ala Gly Ala Ser Ala Ala Leu Ala Leu Thr Ala Ala Leu Ala
        35                  40                  45

Ala Val Pro Val Ala Ala Pro Ala Ala Ala Ala Pro Pro Val Pro Thr
    50                  55                  60

Pro Val Val Asp Ser Pro Asn Gly Arg Gly Asp Val Ile Leu Asn Leu
65                  70                  75                  80

Phe Gln Trp Thr Trp Asp Ser Val Ala Ala Glu Cys Thr Ser Thr Ile
                85                  90                  95

Gly Pro Ala Gly Phe Gly Tyr Val Gln Val Ser Pro Pro Gln Glu His
            100                 105                 110

Ile Gln Gly Thr Ala Trp Trp Thr Ser Tyr Gln Pro Val Ser Tyr Lys
        115                 120                 125

Leu Glu Ser Lys Leu Gly Thr Arg Ala Glu Phe Gln Arg Met Val Ser
    130                 135                 140

Thr Cys Lys Ala Ala Gly Val Gly Val Ile Val Asp Ala Val Val Asn
145                 150                 155                 160

His Thr Ala Gly Ala Asp Thr Gly Ser Gly Thr Gly Thr Gly Gly Thr
                165                 170                 175

Ser Tyr Ser Val Asp Ser Phe Pro Gly Val Pro Tyr Gly Pro Asn Asp
            180                 185                 190

Phe Asn Asp Cys Arg Ser Asn Ile Ser Asn Tyr Gly Asp Arg Tyr Asn
        195                 200                 205

Val Gln Asn Cys Arg Leu Val Ser Leu Gln Asp Leu Arg Thr Gly Ser
    210                 215                 220

Asp Tyr Val Arg Asp Lys Ile Ala Gly Tyr Leu Asn Asp Leu Leu Ser
225                 230                 235                 240

Leu Gly Val Ser Gly Phe Arg Ile Asp Ala Ala Lys His Ile Pro Ala
                245                 250                 255

Ala Asp Leu Ala Ala Ile Lys Ala Arg Leu Thr Asn Pro Asp Val Phe
            260                 265                 270

Trp Val His Glu Val Ile Gly Ala Ser Gly Glu Pro Ile Gln Pro Ser
        275                 280                 285

Glu Tyr Leu Gly Ser Gly Asp Ser His Glu Phe Phe Tyr Ala Arg Glu
    290                 295                 300

Leu Lys Ser Arg Phe Asp Gly Gln Ile Lys Asp Leu Arg Thr Ile Gly
305                 310                 315                 320

Asp Asn Lys Leu Pro Ser Asp Arg Ala Gly Val Phe Val Asp Asn His
                325                 330                 335

Asp Thr Glu Arg Asn Gly Glu Thr Met Ser Tyr Lys Trp Gly Ala Lys
            340                 345                 350

Tyr Arg Leu Ala Asn Ala Phe Met Leu Ser Trp Pro Tyr Gly Ala Pro
        355                 360                 365

Ser Val Tyr Ser Gly Tyr Thr Trp Ser Asp Lys Asp Ala Gly Ala Pro
    370                 375                 380

Gly Ala Ser Ala Thr Ser Val Pro Asp Ala Ser Cys Ala Asp Ser Ala

385                 390                 395                 400

Trp Val Cys Thr Gln Arg Trp Thr Glu Ile Ala Gly Met Val Gly Phe
                405                 410                 415

His Asn Ala Val Ala Gly Thr Pro Val Thr Gly Trp Trp Asp Asp Gly
            420                 425                 430

Asn Asn His Ile Ala Tyr Gly Arg Gly Ala Lys Gly Tyr Val Thr Ile
        435                 440                 445

Asn Asn Ser Ala Asn Ala Val Thr Arg Thr Tyr Gln Thr Ser Leu Pro
    450                 455                 460

Ala Gly Thr Tyr Cys Asp Val Val Ala Ser Lys Asp Cys Ser Lys Thr
465                 470                 475                 480

Val Ser Val Ser Gly Ser Gly Thr Phe Thr Ala Thr Val Pro Ala Tyr
                485                 490                 495

Gly Ala Leu Ala Leu His Val Gly Ala Arg Gly Asp Gly Gly Thr Gly
            500                 505                 510

Gly Pro Gly Pro Val Asp Pro Ala Gly Thr Thr Thr Val Tyr Tyr Ala
        515                 520                 525

Thr Asp Lys Gly Trp Asn Ala Tyr Asn Val His Tyr Lys Val Gly Thr
    530                 535                 540

Gly Ala Trp Thr Ala Val Pro Gly Val Pro Met Thr Ala Ala Cys Thr
545                 550                 555                 560

Gly Trp Val Ser Arg Gln Ile Ser Leu Pro Ser Gly Ala Thr Gly Ala
                565                 570                 575

Ala Ala Thr Val Thr Ala Ala Phe Thr Asn Gly Ser Gly Ala Trp Asp
            580                 585                 590

Ser Asn Gly Gly Lys Asp Tyr Ser Leu Ser Gly Ala Ala Val Ala Val
        595                 600                 605

Ser Gly Gly Thr Val Thr Ala Gly Asn Pro Cys Asp Gly Gly Gly Ser
    610                 615                 620

Pro Gly Gly Pro Val Glu Gly Gln Gly Asp Ala Ser Phe Ser Val Thr
625                 630                 635                 640

Ala Thr Thr Thr Trp Gly Gln Thr Val His Val Val Gly Ser Ile Pro
                645                 650                 655

Ala Leu Gly Ser Trp Ala Pro Ala Asn Gly Val Pro Leu Ser Ser Ser
            660                 665                 670

Ala Tyr Pro Val Trp Thr Ala Arg Val Asp Leu Pro Ala Gly Thr Thr
        675                 680                 685

Phe Gln Tyr Lys Tyr Val Lys Arg Asp Gly Gly Gly Asn Val Val Trp
    690                 695                 700

Glu Ser Gly Gly Asn Arg Thr Ala Thr Val Gly Ala Asp Gly Ser Val
705                 710                 715                 720

Thr Leu Ser Asp Thr Trp Arg Ser
                725

<210>  4
<211>  1599
<212>  DNA
<213>  Cellulosimicrobacterium.sp

<400>  4
gcacccgccg cggccgcgcc gcccgtgccg acgcccgtcg tcgactcccc gaacggccgg      60

ggcgacgtga tcctcaacct cttccagtgg acgtgggact ccgtggccgc cgagtgcacg     120

agcaccatcg gcccggccgg gttcggctac gtccaggtct cgccgccgca ggagcacatc     180

cagggcacgg cctggtggac ctcctaccag cccgtcagct acaagctcga gtccaagctc     240

ggcacccgcg ccgagttcca gcgcatggtg agcacgtgca aggcggcggg cgtgggcgtg     300

atcgtcgacg cggtcgtcaa ccacacggcg ggcgccgaca ccgggtccgg aaccgggacc     360

ggagggacga gctactccgt ggacagcttc cccggcgtcc cgtacggccc gaacgacttc     420

aacgactgcc ggtcgaacat cagcaactac ggcgaccggt acaacgtcca gaactgccgc     480

ctcgtctcgc tccaggatct gcggacgggc tccgactacg tgcgcgacaa gatcgcgggc     540

tacctcaacg acctcctgtc gctcggcgtc tcgggcttcc gcatcgacgc cgcgaagcac     600

atcccggccg cggacctcgc ggcgatcaag gcgcggctca cgaacccgga cgtcttctgg     660

gtgcacgagg tcatcggcgc gagcggcgag ccgatccagc cctcggagta cctcggttcc     720

ggcgactcgc acgagttctt ctacgcgcgc gagctcaagt cccggttcga cggccagatc     780

aaggacctgc gcaccatcgg cgacaacaag ctcccctccg accgcgcggg cgtcttcgtc     840

gacaaccacg acaccgagcg caacggcgag acgatgagct acaagtgggg cgccaagtac     900

cggctcgcca acgcgttcat gctctcgtgg ccctacggcg cgccgagcgt ctactcgggc     960

tacacgtgga gcgacaagga cgcgggtgcg cccggcgcct ccgccacctc cgtgcccgac    1020

gcgagctgtg ccgacagcgc gtgggtctgc acgcagcgct ggaccgagat cgcgggcatg 1080

gtcggcttcc acaacgcggt cgccgggacc ccggtgacgg gctggtggga cgacgggaac 1140

aaccacatcg cctacgggcg gggcgccaag ggctacgtga cgatcaacaa ctccgcgaac 1200

gccgtgacgc gcacctacca gacctcgctc cccgccggga cgtactgcga cgtcgtggcg 1260

tcgaaggact gctcgaagac cgtctccgtc tccggctcgg gcaccttcac ggcgacggtg 1320

cccgcgtacg gggcgctcgc gctccacgtc ggggcgcgcg gcgacggcgg caccggtggc 1380

cccgggcccg tcgacccggc cggcacgacg accgtctact acgcgaccga caagggctgg 1440

aacgcgtaca acgtccacta caaggtcggc accggcgcct ggaccgccgt ccccggggtc 1500

ccgatgacgg cggcctgcac gggctgggtc tcgcggcaga tcagccttcc cagcggggcg 1560

acgggcgcgg ccgcgaccgt caccgccgcc ttcacgaac 1599

<210> 5
<211> 2028
<212> DNA
<213> Cellulosimicrobacterium.sp

<400> 5
gcacccgccg cggccgcgcc gcccgtgccg acgcccgtcg tcgactcccc gaacggccgg 60

ggcgacgtga tcctcaacct cttccagtgg acgtgggact ccgtggccgc cgagtgcacg 120

agcaccatcg gcccggccgg gttcggctac gtccaggtct cgccgccgca ggagcacatc 180

cagggcacgg cctggtggac ctcctaccag cccgtcagct acaagctcga gtccaagctc 240

ggcacccgcg ccgagttcca gcgcatggtg agcacgtgca aggcggcggg cgtgggcgtg 300

atcgtcgacg cggtcgtcaa ccacacggcg ggcgccgaca ccgggtccgg aaccggaccc 360

ggagggacga gctactccgt ggacagcttc cccggcgtcc cgtacgggcc gaacgacttc 420

aacgactgcc ggtcgaacat cagcaactac ggcgaccggt acaacgtcca gaactgccgc 480

ctcgtctcgc tccaggatct gcggacgggc tccgactacg tgcgcgacaa gatcgcgggc 540

tacctcaacg acctcctgtc gctcggcgtc tcgggcttcc gcatcgacgc cgcgaagcac 600

atcccggccg cggacctcgc ggcgatcaag gcgcggctca cgaacccgga cgtcttctgg 660

gtgcacgagg tcatcggcgc gagcggcgag ccgatccagc cctcggagta cctcggttcc 720

ggcgactcgc acgagttctt ctacgcgcgc gagctcaagt cccggttcga cggccagatc 780

aaggacctgc gcaccatcgg cgacaacaag ctcccctccg accgcgcggg cgtcttcgtc 840

gacaaccacg acaccgagcg caacggcgag acgatgagct acaagtgggg cgccaagtac 900

cggctcgcca acgcgttcat gctctcgtgg ccctacggcg cgccgagcgt ctactcgggc 960

tacacgtgga gcgacaagga cgcgggtgcg cccggcgcct ccgccacctc cgtgcccgac 1020

gcgagctgtg ccgacagcgc gtgggtctgc acgcagcgct ggaccgagat cgcgggcatg 1080

gtcggcttcc acaacgcggt cgccgggacc ccggtgacgg gctggtggga cgacgggaac 1140

aaccacatcg cctacggcgg gggcgccaag ggctacgtga cgatcaacaa ctccgcgaac 1200

gccgtgacgc gcacctacca gacctcgctc cccgccggga cgtactgcga cgtcgtggcg 1260

tcgaaggact gctcgaagac cgtctccgtc tccggctcgg gcaccttcac ggcgacggtg 1320

cccgcgtacg gggcgctcgc gctccacgtc ggggcgcgcg gcgacggcgg caccggtggc 1380

cccgggcccg tcgacccggc cggcacgacg accgtctact acgcgaccga caagggctgg 1440

aacgcgtaca acgtccacta caaggtcggc accggcgcct ggaccgccgt ccccggggtc 1500

ccgatgacgg cggcctgcac gggctgggtc tcgcggcaga tcagccttcc cagcggggcg 1560

acgggcgcgg ccgcgaccgt caccgccgcc ttcacgaacg gctcgggcgc ctgggacagc 1620

aacggcggca aggactactc cctgtccggc gctgcggtcg ccgtctccgg cgggacggtc 1680

acggcgggca acccgtgcga cggcggcggg tcgccgggcg ggccggtcga agggcagggc 1740

gacgcgtcgt tctccgtcac cgccaccacc acgtggggcc agacggtcca cgtcgtcggc 1800

agcatcccgg cgctcggctc ctgggcgccc gcgaacggcg tcccgctgtc gtcgtcggcc 1860

tacccggtgt ggaccgcgcg cgtcgacctc ccggccggca cgacgttcca gtacaagtac 1920

gtcaagcgcg acggcggcgg gaacgtcgtc tgggagagcg gcggcaaccg cacggcgacg 1980

gtcggcgccg acggctccgt gacgctgtcg gacacctggc ggtcgtag 2028

<210> 6
<211> 2187
<212> DNA
<213> Cellulosimicrobacterium.sp

<400> 6
atgacgcgca ccaccgacct cgccggaccc ccggcgccca cccccgtccc cgcaggacgc 60

cccgcccgcc cgcgcggccg ccgcttgcgc cgcgtcctcg cggccggcgc ctcggcggcc 120

ctcgcgctga ccgccgcgct ggccgcggtc ccggtcgccg cacccgccgc ggccgcgccg 180

cccgtgccga cgcccgtcgt cgactccccg aacggccggg gcgacgtgat cctcaacctc 240

ttccagtgga cgtgggactc cgtggccgcc gagtgcacga gcaccatcgg cccggccggg 300

ttcggctacg tccaggtctc gccgccgcag gagcacatcc agggcacggc ctggtggacc 360

tcctaccagc ccgtcagcta caagctcgag tccaagctcg gcacccgcgc cgagttccag 420

cgcatggtga gcacgtgcaa ggcggcgggc gtgggcgtga tcgtcgacgc ggtcgtcaac 480

cacacggcgg gcgccgacac cgggtccgga accgggaccg gagggacgag ctactccgtg 540

gacagcttcc ccggcgtccc gtacgggccg aacgacttca acgactgccg gtcgaacatc 600

agcaactacg gcgaccggta caacgtccag aactgccgcc tcgtctcgct ccaggatctg 660

cggacgggct ccgactacgt gcgcgacaag atcgcgggct acctcaacga cctcctgtcg 720

ctcggcgtct cgggcttccg catcgacgcc gcgaagcaca tcccggccgc ggacctcgcg 780

gcgatcaagg cgcggctcac gaacccggac gtcttctggg tgcacgaggt catcggcgcg 840

agcggcgagc cgatccagcc ctcggagtac ctcggttccg gcgactcgca cgagttcttc 900

tacgcgcgcg agctcaagtc ccggttcgac ggccagatca aggacctgcg caccatcggc 960

gacaacaagc tcccctccga ccgcgcgggc gtcttcgtcg acaaccacga caccgagcgc 1020

aacggcgaga cgatgagcta caagtggggc gccaagtacc ggctcgccaa cgcgttcatg 1080

ctctcgtggc cctacggcgc gccgagcgtc tactcgggct acacgtggag cgacaaggac 1140

gcgggtgcgc ccggcgcctc cgccacctcc gtgcccgacg cgagctgtgc cgacagcgcg 1200

tgggtctgca cgcagcgctg gaccgagatc gcgggcatgg tcggcttcca caacgcggtc 1260

gccgggaccc cggtgacggg ctggtgggac gacgggaaca accacatcgc ctacgggcgg 1320

ggcgccaagg gctacgtgac gatcaacaac tccgcgaacg ccgtgacgcg cacctaccag 1380

acctcgctcc ccgccgggac gtactgcgac gtcgtggcgt cgaaggactg ctcgaagacc 1440

gtctccgtct ccggctcggg caccttcacg gcgacggtgc ccgcgtacgg ggcgctcgcg 1500

ctccacgtcg gggcgcgcgg cgacggcggc accggtggcc ccgggcccgt cgacccggcc 1560

ggcacgacga ccgtctacta cgcgaccgac aagggctgga acgcgtacaa cgtccactac 1620

aaggtcggca ccggcgcctg gaccgccgtc cccggggtcc cgatgacggc ggcctgcacg 1680

ggctgggtct cgcggcagat cagccttccc agcggggcga cgggcgcggc cgcgaccgtc 1740

accgccgcct tcacgaacgg ctcgggcgcc tgggacagca acggcggcaa ggactactcc 1800

ctgtccggcg ctgcggtcgc cgtctccggc gggacggtca cggcgggcaa cccgtgcgac 1860

ggcggcgggt cgccgggcgg gccggtcgaa gggcagggcg acgcgtcgtt ctccgtcacc 1920

gccaccacca cgtggggcca gacggtccac gtcgtcggca gcatcccggc gctcggctcc 1980

tgggcgcccg cgaacggcgt cccgctgtcg tcgtcggcct acccggtgtg gaccgcgcgc 2040

gtcgacctcc cggccggcac gacgttccag tacaagtacg tcaagcgcga cggcggcggg 2100

aacgtcgtct gggagagcgg cggcaaccgc acggcgacgg tcggcgccga cggctccgtg 2160

acgctgtcgg acacctggcg gtcgtag 2187

<210> 7
<211> 35
<212> DNA
<213> artificial

<220>
<223> primer

<400> 7
ggcagtctgc tcatgcgatg acgcgcacca ccgac 35

<210>  8
<211>  33
<212>  DNA
<213>  artificial

<220>
<223>  primer

<400>  8
ttacttttta ttcagttacg accgccaggt gtc                                    33